# EUROPÄISCHE PATENTSCHRIFT

(11) **EP 0 739 880 B1**
(45) Veröffentlichungstag und Bekanntmachung des Hinweises auf die Patenterteilung: **31.03.1999**
(21) Anmeldenummer: 96106068.8
(22) Anmeldetag: 18.04.1996
(51) Int. Cl.: C07C 409/40

(54) **Verfahren zur Herstellung von Amidoperoxicarbonsäuren**
Process for the preparation of amido peroxycarboxylic acids
Procédé pour la préparation d'acides amido peroxycarboxyliques

(30) Priorität: 27.04.1995 DE 19515443
(43) Veröffentlichungstag der Anmeldung: 30.10.1996
(73) Patentinhaber: Clariant GmbH, 65929 Frankfurt am Main (DE)
(72) Erfinder: Löffler, Matthias, Dr., 65527 Niedernhausen (DE)

(56) Entgegenhaltungen:
- EP-A- 0 485 928
- EP-A- 0 564 251

## Beschreibung

Die britische Patentschrift Nr. 855 735 offenbart Bleichmittel enthaltend ein anorganisches Persalz, wie Perborat, Percarbonat, Perhydrophosphat und Persilikat und ein reaktives organisches Acylamid, z.B. N-Acyllactam, wie N-Acetylcaprolactam.
Der Nachteil dieser Bleichmittel liegt darin, daß die erforderliche Reaktivität der einzusetzenden organischen Acylamide von bestimmten Moleküleigenschaften abhängig ist und damit keine genaue Voraussage über den Einsatz eines Acylamids gemacht werden kann.
Ein weiterer Gegenstand dieser Patentschrift sind Reinigungsmittel, die als Bleichmittel eine Amidoperoxicarbonsäure enthalten.

EP-A-0 564 250 betrifft Reinigungsflüssigkeiten, die Amidoperoxicarbonsäuren als Bleichmittel enthalten, wobei Amido-mono-peroxicarbonsäuren und Amido-di-peroxicarbonsäuren als bevorzugte Persäuren genannt werden. Die Herstellung der zugrundeliegenden Amidocarbonsäuren erfolgt unter den Bedingungen der Schotten-Baumann-Reaktion, d.h. durch Umsetzung eines Amins mit einem Säurechlorid zu dem entsprechenden Carbonsäureamid. Der in diesem Zusammenhang gewählte Begriff des "Amins" ist weit gefasst und beinhaltet sowohl Amine, wie Piperazin und Anilin aber auch Aminosäuren, wie die Aminobenzoesäure. Durch Umsetzung dieser Amine mit den gewünschten Säurechloriden werden dann Amido-mono- bzw. Amido-di-carbonsäuren erhalten, die in einer weiteren Umsetzung mit einem Gemisch aus Methansulfonsäure und Wasserstoffperoxid zu den entsprechenden Amidoperoxicarbonsäuren oxidiert werden.
Nachteilig an diesem Herstellungsverfahren ist der erforderliche Einsatz von Säurechloriden, der verfahrensbedingte Salzanfall sowie die teilweise unzureichende Ausbeute an Amidoperoxicarbonsäure.

Aufgabe der vorliegenden Erfindung ist es, ein verbessertes Verfahren zur Herstellung von Amidoperoxicarbonsäuren zur Verfügung zu stellen.

Gegenstand der vorliegenden Erfindung ist ein Verfahren zur Herstellung von Amidoperoxicarbonsäuren umfassend die Verfahrensschritte:
A) Ringöffnung des N-Acyllactams zu der entsprechenden Amidocarbonsäure und nachfolgend
B) Oxidation der erhaltenen Amidocarbonsäure zur entsprechenden Amidoperoxicarbonsäure.

Bei den Amidoperoxicarbonsäuren handelt es sich bevorzugt um Mono- oder Diamidoperoxicarbonsäuren.
Monoamidoperoxicarbonsäuren besitzen bevorzugt die allgemeine Formel 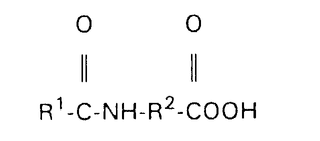 worin
- R¹: C₁-C₂₀-Alkyl, C₂-C₂₀-Alkenyl, C₅-C₂₀-Cycloalkyl, C₆-C₁₈-Aryl oder C₁-C₂₀-Alkyl-C₆-C₁₈-Aryl und
- R²: C₁-C₂₀-Alkylen, C₂-C₂₀-Alkenylen, C₅-C₂₀-Cycloalkylen, C₆-C₁₀-Arylen oder C₁-C₂₀-Alkyl-C₆-C₁₈-Arylen
bedeuten.

Diamidoperoxicarbonsäuren besitzen bevorzugt die allgemeine Formel 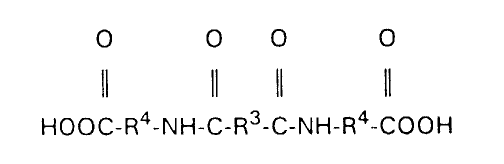 worin
- R³ und R⁴: unabhängig voneinander C₁-C₂₀-Alkylen, C₂-C₂₀-Alkenylen, C₅-C₂₀-Cycloalkylen, C₆-C₁₈-Arylen oder C₁-C₂₀-Alkyl-C₆-C₁₈-Arylen bedeuten.

Besonders bevorzugte Amidoperoxicarbonsäuren sind:
N,N'-Di-(4-Percarboxybenzoyl)-1,4-butandiamin;
N,N'-Di-(4-Percarboxybenzoyl)-1,2-phenylendiamin;
N,N'-Succinoyl-di-(4-percarboxy)-anilin;
4-Percarboxybenzoyl-anilin;
N,N-Phthaloyl-4-aminoperbenzoesäure;
N,N'-Di-(4-Percarboxybenzoyl)-ethylendiamin;
N,N'-Di-(4-Percarboxyanilin)-terephthalat;
N,N,N',N'-1,2,4,5-Tetracarboxybenzoyl-di-(6-aminopercarboxycapronsäure);
N,N'-Di-(4-Percarboxybenzoyl)-piperazin;
N,N'-Di-(4-Percarboxybenzoyl)-1,4-diamin-cyclohexan;
N,N'-Di-(Percarboxyadipoyl)-phenylendiamin;
N,N'-Di-(Percarboxyadipoyl)-ethylendiamin;
N,N'-Terephthaloyl-di-(6-aminoperoxicapronsäure);
N,N'-Terephthaloyl-di-(12-aminoperoxilaurinsäure) und
N,N'-Oxalyl-di-(6-aminoperoxicapronsäure).

Bei dem erfindungsgemäßen Verfahren erfolgt zunächst die Ringöffnung des N-Acyllactams zu der entsprechenden Amidocarbonsäure.

Sofern die Acyllactame als Ausgangsstoffe nicht verfügbar sind, lassen sich diese durch Umsetzung von Lactamen mit reaktiven Carbonylverbindungen leicht herstellen. Die in der Literatur beschriebenen Synthesen betreffen hauptsächlich die Umsetzung von Lactamen mit Anhydriden (GB-A-1 102 521; US-A-4 608 201) bzw. mit Säurechloriden (J. Amer. Chem. Soc. 80 (1958) 6420; J. Org. Chem. 29 (1964) 2425 und Bull. Chem. Soc. Jpn. 8 (1964) 1245).

Die nachfolgende Aufstellung gibt einen Überblick über handelsübliche Lactame und deren N-Acyllactame:
1. N-unsubstituierte Lactame
   2-Azetidinon
   2-Pyrrolidinon
      3-Methyl-2-pyrrolidinon
      5-Methyl-2-pyrrolidinon
   2-Piperidon
      6-Methyl-2-piperidon
   ε-Caprolactam
      α-Dimethylamino-ε-carprolactam
      α-Amino-ε-caprolactam
      7-Butyl-ε-caprolactam
      3,3-Dibromo-ε-caprolactam
   2-Azacyclooctanon
   2-Azacyclononanon
   2-Azacyclodecanon
2. N-Acetyl-Lactame
   N-Acetyl-2-azetidinon
   N-Acetyl-2-pyrrolidinon
   N-Acetyl-3-methyl-2-pyrrolidinon
      N-Acetyl-5-methyl-2-pyrrolidinon
   N-Acetyl-2-piperidon
      N-Acetyl-6-methyl-2-piperidon
   N-Acetyl-ε-caprolactam
      N-Acetyl-α-dimethylamino-ε-caprolactam
      N-Acetyl-α-amino-ε-caprolactam
      N-Acetyl-ε-caprolactam
      N-Acetyl-3,3-dibromo-ε-caprolactam
   N-Acetyl-2-azacyclooctanon
   N-Acetyl-2-azacyclononanon
   N-Acetyl-2-azacyclodecanon
3. N-Benzoyl-Lactame
   N-Benzoyl-2-azetidinon
   N-Benzoyl-2-pyrrolidinon
   N-Benzoyl-3-methyl-2-pyrrolidinon
      N-Benzoyl-5-methyl-2-pyrrolidinon
   N-Benzoyl-2-piperidon
      N-Benzoyl-6-methyl-2-piperidon
   N-Benzoyl-ε-caprolactam
      N-Benzoyl-α-dimethylamino-ε-caprolactam
      N-Benzoyl-α-amino-ε-caprolactam
      N-Benzoyl-7-butyl-ε-caprolactam
      N-Benzoyl-3,3-dibromo-ε-caprolactam
   N-Benzoyl-2-azacyclooctanon
   N-Benzoyl-2-azacyclononanon
   N-Benzoyl-2-azacyclodecanon
4. Weitere N-Alkanoyl-Lactame
   N-Octanoyl-2-pyrrolidinon
      N-Octanoyl-3-methyl-2-pyrrolidinon
      N-Octanoyl-5-methyl-2-pyrrolidinon
   N-Octanoyl-2-piperidon
      N-Octanoyl-6-methyl-2-piperidon
   N-Octanoyl-ε-caprolactam
      N-Octanoyl-α-amino-ε-caprolactam
      N-Octanoyl-3,3-dibromo-ε-caprolactam
   N-Octanoyl-2-azacyclooctanon
   N-Octanoyl-2-azacyclononanon
   N-Octanoyl-2-azacyclodecanon
   N-Decanoyl-2-pyrrolidinon
      N-Decanoyl-3-methyl-2-pyrrolidinon
      N-Decanoyl-5-methyl-2-pyrrolidinon
   N-Decanoyl-2-piperidon
      N-Decanoyl-6-methyl-2-piperidon
   N-Decanoyl-ε-caprolactam
      N-Decanoyl-α-amino-ε-caprolactam
      N-Decanoyl-3,3-dibromo-4,5,6,7-tetrahydro-1H-azepin-2(3H)-on
   N-Decanoyl-2-azacyclooctanon
   N-Decanoyl-2-azacyclononanon
   N-Decanoyl-2-azacyclodecanon
5. Bifunktionelle N-Acyl-Lactame
   N,N'-Terephthaloylbis-2-azetidion
   N,N'-Terephthaloylbis-2-pyrrolidinon
   N,N'-Terephthaloylbis-2-piperidon
   N,N'-Terephthaloylbis-caprolactam
   N,N'-Terephthaloylbis-2-azacyclooctanon
   N,N'-Terephthaloylbis-2-azacyclononanon
   N,N'-Terephthaloylbis-2-azacyclodecanon

Gemäß der vorliegenden Erfindung erfolgt die Ringöffnung der N-Acyllactame üblicherweise durch Umsetzung in der wäßrigen Lösung einer starken Säure. Als starke Säure eignen sich bevorzugt Schwefelsäure und Methansulfonsäure. Die Menge der Säure beträgt bevorzugt das ein- bis achtfache, besonders bevorzugt das drei- bis siebenfache der Gewichtsmenge an N-Acyllactam.
Die zur Ringöffnung eingesetzte Menge an Wasser entspricht dem ein- bis zehnfachen molaren Überschuß, bezogen auf die Carbonylgruppe des N-Acyllactams.
Die Art und Weise wie N-Acyllactam, Säure und Wasser miteinander gemischt werden, ist nicht kritisch.
Werden Säure und Wasser getrennt eingesetzt, so ist es vorteilhaft, das N-Acyllactam der Säure zuzusetzen, wobei vielfach das N-Acyllactam in Lösung geht und nachfolgend das Wasser zuzugeben. Ebenso ist es möglich, N-Acyllactam und Wasser zu mischen, wobei vielfach eine Suspension erhalten wird und nachfolgend die Säure zuzugeben. Entsprechend zweckmäßig ist der Einsatz verdünnter Säure in den vorstehend angegebenen Mengen. Bei Einsatz von Schwefelsäure wird üblicherweise eine 50 bis 96 gew.-%ige, bevorzugt 75 bis 96 gew.-%ige wäßrige Lösung verwendet.

Die für die Ringöffnung erforderlichen Reaktionstemperaturen sind in einem weiten Bereich wählbar und liegen im allgemeinen zwischen 10°C und dem Siedepunkt der Lösung. Bevorzugt wird die Reaktion bei einer Temperatur im Bereich von 20 bis 90°C durchgeführt.

Die bei der Umsetzung des N-Acyllactams mit Säure und Wasser entstehende Amidocarbonsäure wird in vielen Fällen in hoher Ausbeute erhalten. Eine Isolierung, Reinigung oder anderweitige Aufarbeitung kann deshalb entfallen. Eine große Anzahl an Amidocarbonsäuren ist im Reaktionsmedium schlecht löslich und kann durch Filtration isoliert werden sowie gegebenenfalls durch Umkristallisation gereinigt werden. Gerade wegen der verfahrensgemäßen hohen Ausbeute ist es vorteilhaft, die erhaltene Amidocarbonsäure direkt anschließend zur Amidoperoxicarbonsäure zu oxidieren.

Die Oxidation der Amidocarbonsäure erfolgt unter Verwendung der üblichen Oxidationsmittel, bevorzugt Wasserstoffperoxid.
Die Oxidation erfolgt vorzugsweise bei milden Temperaturen, bevorzugt zwischen -5 und 40°C, besonders bevorzugt zwischen 20 und 35°C.
Die eingesetzte Wasserstoffperoxidlösung besitzt handelsübliche Konzentration, bevorzugt 25 bis 85 gew.-%ige Lösung.
Das Oxidationsmittel wird im ein- bis zehnfachen, bevorzugt zwei- bis vierfachen molaren Überschuß, bezogen auf die Carboxylgruppe der Amidocarbonsäure, eingesetzt.

Die derart erhaltenen Amidoperoxicarbonsäuren fallen in hohen Ausbeuten an und lassen sich durch Zugabe von Wasser ausfällen. Durch Zugabe von wäßrigen basischen Salzen lassen sich die entsprechenden Salze erhalten. Durch Umkristallisation ist eine Reinigung möglich.
Die Vorteile des erfindungsgemäßen Verfahrens zeigen sich u.a. in der Vermeidung des Einsatzes von Säurechlorid, der Verringerung des bisher bedingten Salzanfalls und die Herstellung der Amidoperoxicarbonsäure ohne Isolierung der Zwischenprodukte aus Verfahrensschritt (A).

### Herstellungsbeispiele:

### Beispiel 1

### N,N'-Terephthaloylbiscaprolactam

Zu einer Lösung von ε-Caprolactam (56,6 g, 0,5 mol) in 400 ml Cyclohexan und 117 ml Pyridin wird bei Raumtemperatur Terephthaloylchlorid (51 g, 0,25 mol) innerhalb von 30 Minuten zugegeben. Danach wird für 4 h unter Rückfluß erhitzt. Nach Abkühlen wird der resultierende hellgelbe Niederschlag abgesaugt, mit Wasser gewaschen und getrocknet. Ausbeute 81 g (91 %).

### N,N'-Terephthaloyl-di-(6-aminoperoxicapronsäure)

Zu einer 80°C warmen, flüssigen Mischung aus N,N'-Terephthaloylbiscaprolactam (17,9 g, 50 mmol) und Wasser (4,0 g, 220 mmol) werden 70 g konzentrierte Schwefelsäure innerhalb einer Stunde zugetropft. Nachdem die Reaktionslösung auf 20°C abgekühlt ist, werden 25 ml 70 %ige wäßrige Wasserstoffperoxidlösung zugetropft. Die Innentemperatur wird dabei durch Eiskühlung zwischen 30 und 35°C gehalten. Nach einer Stunde werden weitere 5 ml 70%ige Wasserstoffperoxidlösung zugetropft und bei 30 bis 35°C eine Stunde nachgerührt. Der Reaktionsansatz wird anschließend mit 500 ml Eiswasser versetzt, der resultierende farblose Niederschlag abgesaugt und mit Wasser neutral gewaschen. Ausbeute 21,0 g (99 %), Aktivsauerstoffgehalt 6,7 % (89,3 %).

### Beispiel 2

### N,N'-Terephthaloylbislaurinlactam

Zu einer Lösung von ω-Laurinlactam (403 g, 2 mol) in 1600 ml Cyclohexan und 475 ml Pyridin wird bei Raumtemperatur Terephthaloylchlorid (203 g, 1 mol) innerhalb von 30 Minuten zugegeben. Danach wird für 3 h unter Rückfluß erhitzt. Nach Abkühlen wird der Kolbeninhalt in 3 l Eiswasser gegossen und der resultierende hellgelbe Niederschlag abgesaugt und getrocknet. Ausbeute 498 g (95 %).

### N,N'-Terephthaloyl-di-(12-aminoperoxilaurinsäure)

Eine Suspension von N,N'-Terephthaloylbislaurinlactam (26,2 g, 50 mmol) in Wasser (4,0 g, 220 mmol) und konzentrierter Schwefelsäure (100 g) wird bei 25°C 4 h gerührt. Anschließend werden 30 ml 70 %ige wäßrige Wasserstoffperoxidlösung innerhalb einer Stunde zugetropft, die Innentemperatur wird dabei durch Eiskühlung zwischen 30 und 35°C gehalten. Nach einer Stunde Nachrührzeit wird der Reaktionsansatz mit 500 ml Eiswasser versetzt, der resultierende farblose Niederschlag abgesaugt und mit Wasser neutral gewaschen. Ausbeute 27,3 g (98 %), Aktivsauerstoffgehalt 5,0 % (86,5 %).

### Beispiel 3

### N-Benzoyl-6-aminoperoxicapronsäure

Zu einer 68°C warmen, flüssigen Mischung aus N-Benzoylcaprolactam (21,7 g, 100 mmol) und Wasser (8,0 g, 440 mmol) werden 140 g Schwefelsäure innerhalb einer halben Stunde zugetropft. Die Innentemperatur steigt dabei auf 80°C, bei dieser Temperatur wird 1 Stunde nachgerührt. Nachdem die Reaktionslösung auf 20°C abgekühlt ist, werden 42 ml 50 %ige wäßrige Wasserstoffperoxidlösung zugetropft. Die Innentemperatur wird dabei durch Eiskühlung zwischen 25 und 30°C gehalten. Nach einer Stunde wird der Reaktionsansatz mit 500 ml Eiswasser versetzt, der resultierende farblose Niederschlag abgesaugt und mit Wasser neutral gewaschen. Ausbeute 22.2 g (88 %), Aktivsauerstoffgehalt 6,2 % (98 %).

### Beispiel 4

### N,N'-Oxalylbiscaprolactam

Zu einer Lösung von ε-Caprolactam (107,6 g, 0,95 mol) in Toluol (300 ml) wird Oxalsäuredichlorid (63,4 g, 0,5 mol) innerhalb von 30 Minuten zugetropft. Die Innentemperatur erhöht sich dabei von 20 bis 60°C. Anschließend wird 2 Stunden unter Rückfluß erhitzt. Nach Entfernung des Lösungsmittels im Vakuum wird das resultierende Öl in Methanol aufgenommen, der in der Kälte ausfallende farblose Niederschlag wird isoliert und getrocknet. Ausbeute 113,8 g (86 %).

### N,N'-Oxalyl-di-(6-aminoperoxicapronsäure)

Eine Suspension von N,N'-Oxalylbiscaprolactem (14,17 g, 50 mmol) in Wasser (1,8 g, 100 mmol) und konzentrierter Schwefelsäure (50 g) wird bei Raumtemperatur vier Stunden gerührt. Danach wird unter Eiskühlung 50 gew.-%ige Wasserstoffperoxidlösung (40,8 g) so zugetropft, daß die Innentemperatur 25°C nicht überschreitet. Nach einer Stunde Rühren bei 30°C wird Wasser (250 ml) zugegeben, der resultierende farblose Niederschlag wird abgesaugt, mit Wasser neutral gewaschen und getrocknet. Ausbeute 15,5 g, (89 %), Aktivsauerstoff 8,8 % (96 %).

## Patentansprüche

1. Verfahren zur Herstellung von Amidoperoxicarbonsäuren umfassend die Verfahrensschritte:
A) Ringöffnung des N-Acyllactams zu der entsprechenden Amidocarbonsäure und nachfolgend
B) Oxidation der erhaltenen Amidocarbonsäure zur entsprechenden Amidoperoxicarbonsäure.

2. Verfahren nach Anspruch 2, dadurch gekennzeichnet, daß in Verfahrensschritt (A) die Ringöffnung des N-Acyllactams in der wäßrigen Lösung einer starken Säure erfolgt.

3. Verfahren nach Anspruch 1 oder 2, dadurch gekennzeichnet, daß die in Verfahrensschritt (A) eingesetzte Säuremenge das ein- bis achtfache der Gewichtsmenge des N-Acyllactams beträgt.

4. Verfahren nach Anspruch 3, dadurch gekennzeichnet, daß die Säuremenge das drei- bis siebenfache der Gewichtsmenge des N-Acyllactams beträgt.

5. Verfahren nach einem der Ansprüche 1 bis 4, dadurch gekennzeichnet, daß die in Verfahrensschritt (A) eingesetzte Wassermenge dem ein- bis zehnfachen molaren Überschuß, bezogen auf die Carbonylgruppe des N-Acyllactams, entspricht.

6. Verfahren nach einem der Ansprüche 1 bis 5, dadurch gekennzeichnet, daß in Verfahrensschritt (A) Methansulfonsäure oder Schwefelsäure eingesetzt wird.

7. Verfahren nach einem der Ansprüche 1 bis 6, dadurch gekennzeichnet, daß in Verfahrensschritt (A) 50 bis 96 gew.-%ige wäßrige Schwefelsäure eingesetzt wird.

8. Verfahren nach einem der Ansprüche 1 bis 7, dadurch gekennzeichnet, daß in Verfahrensschritt (A) die Ringöffnung bei einer Reaktionstemperatur zwischen 10°C und dem Siedepunkt der Lösung erfolgt.

9. Verfahren nach Anspruch 8, dadurch gekennzeichnet, daß die Reaktionstemperatur im Bereich von 20 bis 90°C liegt.

10. Verfahren nach einem der Ansprüche 1 bis 9, dadurch gekennzeichnet, daß in Verfahrensschritt (B) die Oxidation der Amidocarbonsäure mittels Wasserstoffperoxid erfolgt.

11. Verfahren nach Anspruch 10, dadurch gekennzeichnet, daß eine 25 bis 85 gew.-%ige wäßrige Wasserstoffperoxidlösung eingesetzt wird.

12. Verfahren nach einem der Ansprüche 1 bis 11, dadurch gekennzeichnet, daß die Oxidation bei einer Temperatur zwischen -5 und 40°C durchgeführt wird.

13. Verfahren nach Anspruch 12, dadurch gekennzeichnet, daß die Temperatur zwischen 20 und 35°C beträgt.

14. Verfahren nach einem der Ansprüche 1 bis 13, dadurch gekennzeichnet, daß Verfahrensschritt (B) im Anschluß an Verfahrensschritt (A) ohne Isolierung der Amidocarbonsäure erfolgt.

## Claims

1. A process for the preparation of amidoperoxycarboxylic acids, comprising the process steps:
A) ring opening of the N-acyllactam to give the corresponding amidocarboxylic acid and, subsequently,
B) oxidation of the resulting amidocarboxylic acid to the corresponding amidoperoxycarboxylic acid.

2. The process as claimed in claim 1, wherein, in process step (A), the N-acyllactam ring is opened in the aqueous solution of a strong acid.

3. The process as claimed in claim 1 or 2, wherein the amount of acid used in process step (A) is one to eight times the amount by weight of the N-acyllactam.

4. The process as claimed in claim 3, wherein the amount of acid is three to seven times the amount by weight of the N-acyllactam.

5. The process as claimed in one of claims 1 to 4, wherein the amount of water used in process step (A) corresponds to one to ten times the molar excess, based on the carbonyl group of the N-acyllactam.

6. The process as claimed in one of claims 1 to 5, wherein, in process step (A), methanesulfonic acid or sulfuric acid is used.

7. The process as claimed in one of claims 1 to 6, wherein, in process step (A), 50 to 96 % strength by weight aqueous sulfuric acid is used.

8. The process as claimed in one of claims 1 to 7, wherein, in process step (A), the ring is opened at a reaction temperature between 10 C and the boiling point of the solution.

9. The process as claimed in claim 8, wherein the reaction temperature is in the range from 20 to 90 C.

10. The process as claimed in one of claims 1 to 9, wherein, in process step (B), the amidocarboxylic acid is oxidized by hydrogen peroxide.

11. The process as claimed in claim 10, wherein a 25 to 85 % strength by weight aqueous hydrogen peroxide solution is used.

12. The process as claimed in one of claims 1 to 11, wherein the oxidation is carried out at a temperature between -5 and 40 C.

13. The process as claimed in claim 12, wherein the temperature is between 20 and 35 C.

14. The process as claimed in one of claims 1 to 13, wherein process step (B) is carried out subsequently to process step (A) without isolation of the amidocarboxylic acid.

## Revendications

1. Procédé de préparation d'acides amidoperoxycarboxyliques comprenant les étapes de procédé consistant à :
A) ouvrir le cycle du N-acyllactame pour donner l'acide amidocarboxylique correspondant et ensuite
B) oxyder l'acide amidocarboxylique obtenu pour obtenir l'acide amidoperoxycarboxylique correspondant.

2. Procédé selon la revendication 1, caractérisé en ce que l'on effectue l'ouverture du cycle du N-acyllactame à l'étape de procédé (A) dans la solution aqueuse d'un acide fort.

3. Procédé selon la revendication 1 ou 2, caractérisé en ce que la quantité d'acide utilisée à l'étape de procédé (A) représente une à huit fois la quantité en poids du N-acyllactame.

4. Procédé selon la revendication 3, caractérisé en ce que la quantité d'acide représente trois à sept fois la quantité en poids de N-acyllactame.

5. Procédé selon l'une des revendications 1 à 4, caractérisé en ce que la quantité d'eau utilisée à l'étape de procédé (A) correspond, par rapport au groupe carbonyle du N-acyllactame, à un excès molaire d'une à dix fois.

6. Procédé selon l'une des revendications 1 à 5, caractérisé en ce que l'on utilise à l'étape de procédé (A) l'acide méthanesulfonique ou l'acide sulfurique.

7. Procédé selon l'une des revendications 1 à 6, caractérisé en ce que l'on utilise à l'étape de procédé (A), une solution aqueuse d'acide sulfurique de concentration 50 à 96% en poids.

8. Procédé selon l'une des revendications 1 à 7, caractérisé en ce que l'on réalise à l'étape de procédé (A), l'ouverture du cycle à une température de réaction entre 10°C et le point d'ébullition de la solution.

9. Procédé selon la revendication 8, caractérisé en ce que la température de réaction se situe dans l'intervalle de 20 à 90°C.

10. Procédé selon l'une des revendications 1 à 9, caractérisé en ce qu'à l'étape de procédé (B), on effectue l'oxydation de l'acide amidocarboxylique au moyen de peroxyde d'hydrogène.

11. Procédé selon la revendication 10, caractérisé en ce que l'on utilise une solution aqueuse de peroxyde d'hydrogène de concentration entre 25 et 85% en poids.

12. Procédé selon l'une des revendications 1 à 11, caractérisé en ce que l'on effectue l'oxydation à une température entre -5 et 40°C.

13. Procédé selon la revendication 12, caractérisé en ce que la température est située entre 20 et 35°C.

14. Procédé selon l'une des revendications 1 à 13, caractérisé en ce que l'on réalise l'étape de procédé (B) en liaison avec l'étape de procédé (A) sans isoler l'acide amidocarboxylique.
